# EUROPÄISCHE PATENTSCHRIFT

(11) **EP 1 532 169 B1**
(45) Veröffentlichungstag und Bekanntmachung des Hinweises auf die Patenterteilung: **19.12.2012**
(21) Anmeldenummer: 03793667.1
(22) Anmeldetag: 31.07.2003
(51) Int. Cl.: C07K 14/415, A61K 39/36, C12N 15/29, C12N 5/10

(54) **VARIANTEN DES MAJORALLERGENS PHL P 1 AUS LIESCHGRAS**
VARIANTS OF THE MAJOR ALLERGEN PHL P 1 FROM TIMOTHY GRASS
VARIANTS DE L'ALLERGENE MAJEUR PHL P 1 PROVENANT DE FLEOLE DES PRES

(30) Priorität: 19.08.2002 EP 02018157
(43) Veröffentlichungstag der Anmeldung: 25.05.2005
(73) Patentinhaber: Merck Patent GmbH, 64293 Darmstadt (DE)
(72) Erfinder: SUCK, Roland, 22143 Hamburg (DE); FIEBIG, Helmut, 21493 Schwarzenbek (DE); CROMWELL, Oliver, 23911 Schmilau (DE)
(86) Internationale Anmeldenummer: PCT/EP2003/008471
(87) Internationale Veröffentlichungsnummer: WO 2004/022588

(56) Entgegenhaltungen:
- WO-A-02/22679
- SCHRAMM G ET AL: "Allergen engineering: variants of the timothy grass pollen allergen Phl p 5b with reduced IgE-binding capacity but conserved T cell reactivity" JOURNAL OF IMMUNOLOGY, WILLIAMS & WILKINS CO, US, Bd. 162, Nr. 4, 15. Februar 1999 (1999-02-15), Seiten 2406-2414, XP002216586 ISSN: 0022-1767 in der Anmeldung erwähnt
- LAFFER S ET AL: "COMPLEMENTARY DNA CLONING OF THE MAJOR ALLERGEN PHL P I FROM TIMOTHY GRASS (PHLEUM PRATENSE);RECOMBINANT PHL P I INHIBITS IGE BINDING TO GROUP I ALLERGENS FROM EIGHT DIFFERENT GRASS SPECIES" JOURNAL OF ALLERGY AND CLINICAL IMMUNOLOGY, MOSBY - YEARLY BOOK, INC, US, Bd. 94, Nr. 4, Oktober 1994 (1994-10), Seiten 689-698, XP008001053 ISSN: 0091-6749 in der Anmeldung erwähnt
- VRTALA S ET AL: "IMMUNOLOGIC CHARACTERIZATION OF PURIFIED RECOMBINANT TIMOTHY GRASS POLLEN (PHLEUM PRATENSE) ALLERGENS (PHL P 1, PHL P 2, PHL P 5)" JOURNAL OF ALLERGY AND CLINICAL IMMUNOLOGY, MOSBY - YEARLY BOOK, INC, US, Bd. 97, Nr. 3, März 1996 (1996-03), Seiten 781-787, XP000953173 ISSN: 0091-6749 in der Anmeldung erwähnt
- GROBE K ET AL: "Grass group I allergens (Beta-expansins) are novel, papain-related proteinases" EUROPEAN JOURNAL OF BIOCHEMISTRY, BERLIN, DE, Bd. 263, 1999, Seiten 33-40, XP002181033 ISSN: 0014-2956 in der Anmeldung erwähnt
- SUCK ROLAND ET AL: "Bacterially expressed and optimized recombinant Phl p 1 is immunobiochemically equivalent to natural Phl p 1.", BIOCHIMICA ET BIOPHYSICA ACTA NOV 2006 LNKD- PUBMED:17085087, vol. 1764, no. 11, November 2006 (2006-11) , pages 1701-1709, ISSN: 0006-3002

## Beschreibung

Die Erfindung betrifft Varianten des Majorallergens Phl p 1 aus Lieschgras, dadurch gekennzeichnet, daß eine bisher nicht mögliche Herstellung von monomeren, in physiologischen Medien löslichen und stabilen Molekülen mit Hilfe von prokaryontischen Expressionssystemen und deren anschließende Reinigung erfolgen kann.

### Hintergrund der Erfindung

Allergien vom Typ 1 haben weltweite Bedeutung. Bis zu 25% der Bevölkerung von industrialisierten Länder leiden unter Beschwerden wie allergischer Rhinitis, Konjunktivitis oder Bronchialasthma, die durch in der Luft befindliche Allergene (Aeroallergene) unterschiedlicher Herkunft wie Pflanzenpollen, Milben, Katzen oder Hunden hervorgerufen werden. Bis zu 40% dieser Typ 1-Allergiker wiederum zeigen spezifische IgE (Immunoglobulin E)-Reaktivität bei Gräserpollen (Freidhoff et al., 1986, J. Allergy. Clin. Immunol. 78, 1190-201).

Bei den Typ 1-Allergie auslösenden Substanzen handelt es sich um Proteine, Glykoproteine oder Polypeptide. Diese Allergene reagieren nach Aufnahme über die Schleimhäute mit den bei sensibilisierten Personen an der Oberfläche von Mastzellen gebundenen IgE-Molekülen. Werden zwei IgE-Moleküle durch ein Allergen miteinander vernetzt, führt dies zur Ausschüttung von Mediatoren (z.B. Histamin, Prostaglandinen) und Zytokinen durch die Effektorzelle und damit zu den entsprechenden klinischen Symptomen. In Abhängigkeit von der relativen Häufigkeit der Allergiker, die IgE-Antikörper gegen bestimmte Allergene aufweisen, wird zwischen Major-und Minorallergenen unterschieden. Im Fall vom Lieschgras (*Phleum pratense*) sind bislang Phl p 1 (Petersen et al., 1993, J. Allergy Clin. Immunol. 92, 789-796), Phl p 5 (Matthiesen und Löwenstein, 1991, Clin. Exp. Allergy 21, 297-307), Phl p 6 (Petersen et al., 1995, Int. Arch. Allergy Immunol. 108, 49-54) und Phl p 2/3 (Dolecek et al., 1993) als Majorallergene und Phl p 4 (Löwenstein, 1978, Prog. Allergy 25, 1-62) sowie Gruppe 10 und 11 aus *Lolium perenne* (Ansari et. al., 1987, J. Allergy Clin. Immunol. 80, 229-235) als Minorallergene charakterisiert worden.

Als eine der relevantesten Allergengruppen von Gräserpollen wird Gruppe 1 eingestuft (Tamborini, E. et al., Eur. J. Biochem. 1997, 249:886-894), zu der Phl p 1 aus Lieschgras gehört. Zu Phl p 1 weisen die weiteren Vertreter der Gruppe 1 aus anderen Gräsern Homologien von teilweise über 95% auf (Petersen, A., et al., J. Allergy Clin. Immunol. 1995, 95: 987-994). Aufgrund der hohen Homologien treten bei der Sensibilisierung mit einem Gras auch Reaktionen auf die Allergene anderer kreuzreaktiver Spezies auf. Deshalb sind diese Moleküle für entsprechende Diagnose- und Therapieansätze von übergeordneter Bedeutung.

Bei dem therapeutischen Einsatz dieser Allergene nutzt man die Reaktion mit T-Helferzellen, wobei es zu einer Umorientierung der pathologischen TH2-Zellen in den TH1-Typ kommt. Daraus ergibt sich eine Veränderung des Cytokinprofils derart, daß B-Zellen zur Bildung von IgG statt IgE stimuliert werden.

Ein klassischer Ansatz zur wirksamen therapeutischen Behandlung von Allergien stellt die Spezifische Immuntherapie oder Hyposensibilisierung dar (Fiebig, 1995, Allergo J. 4 (6), 336-339, Bousquet et al., 1998, J. Allergy Clin Immunol. 102(4), 558-562). Dabei werden dem Patienten natürliche Allergenextrakte in steigenden Dosen subkutan injiziert.

Allerdings besteht bei dieser Methode die Gefahr von allergischen Reaktionen oder sogar eines anaphylaktischen Schocks. Um diese Risiken zu minimieren, werden innovative Präparate in Form von Allergoiden eingesetzt. Dabei handelt es sich um chemisch modifizierte Allergenextrakte, die deutlich reduzierte IgE-Reaktivität, jedoch identische T-Zell-Reaktivität im Vergleich zum nicht behandelten Extrakt aufweisen (Fiebig, 1995, Allergo J. 4 (7), 377-382).

Eine noch weitergehende Therapieoptimierung wäre mit rekombinant hergestellten Allergenen möglich. Definierte, ggfs. auf individuelle Patienten abgestimmte Cocktails von hochreinen rekombinant hergestellten Allergenen könnten Extrakte aus natürlichen Allergenquellen ablösen, da diese außer den verschiedenen Allergenen eine größere Zahl von immunogenen, aber nicht allergenen Begleitproteinen enthalten.

Realistische Perspektiven, die zu einer sicheren Hyposenibilisierung mit Expressionsprodukten führen können, bieten gezielt mutierte rekombinante Allergene, bei denen IgE-Epitope spezifisch deletiert werden, ohne die für die Therapie essentiellen T-Zell Epitope zu beeinträchtigen (Schramm et al., 1999, J. Immunol. 162, 2406-2414).

Eine weitere Möglichkeit zur therapeutischen Beeinflussung des gestörten Th-Zell-Gleichgewichtes bei Allergikern ist die Behandlung mit expressionsfähiger DNA, die für die relevanten Allergene kodiert. Erste experimentelle Belege für die allergenspezifische Beeinflussung der Immunantwort konnte an Nagern durch Injektion von Allergen-kodierender DNA erbracht werden (Hsu et al., 1996, Nature Medicine 2 (5), 540-544).

Bei Phl p 1 handelt sich um ein Protein aus 240 Aminosäuren und einer N-Glykosylierungstelle. Die Glykosylierungsanteil beträgt 5% des Molekülargewichtes, welches bei dem natürlichen Protein ca. 30-35 kDa beträgt (Petersen et al., Allergy Clin. Immunol. 1995, 95: 987-994; Suck et al., J. Immunol. Meth. 1999, 229:73-80).

Die Nukleinsäuresequenz von Phl p 1 ist bekannt (Laffer et al., J. Allergy Clin Immunol. 1994, 94: 689-698; Petersen et. al., J. Allergy Clin. Immunol., 1995, 95: 987-94) und kann somit zur rekombinanten Herstellung des Moleküls genutzt werden.

Bisherige Versuche das Molekül in bakteriellen oder eukaryontischen Systemen, wie z.B. Hefe, rekombinant derart herzustellen, daß eine stabile monomere Form erhalten wurde, waren jedoch wegen seiner mangelhaften Löslichkeit nicht erfolgreich:
Im Fall der bakteriellen Expression lagert sich Phl p 1 als Einschlußkörper (inclusion bodies) ab (Vrtala et al., J. Allergy Clin. Immunol, 1996; 97: 781-7) und muß vor der Reinigung zunächst denaturiert werden. Im Anschluß wird das Denaturierungsmittel entzogen. Eine vollständige Rückfaltung des Proteins in die natürliche lösliche Konformation konnte allerdings bisher nicht erreicht werden (Andersson, Lidholm, Int. Arch. Allergy Immunol. 2003;130:87-107).
Ein möglicher Hinderungsgrund für die Bildung einer stabilen Konformation hätte das Fehlen der Glykosylierung sein können. Jedoch wurde selbst in eukaryontischen Systemen, in denen Glykosylierung möglich ist, kein stabiles Phl p 1 erhalten (K. Grobe, Dissertation, 1998, Universität Hamburg). Als eine Ursache für die fehlende Löslichkeit wird stattdessen eine proteolytische Aktivität vermutet, die zur Selbstdegradation des Moleküls führt (Grobe et al., Eur. J. Biochem. 1999; 263: 33-40; Kirsten Gehlhar, Dissertation, 1998, Medizinische Universität zu Lübeck, Deutschland). Daneben kommen auch hydrophobe Interaktionen zwischen den Molekülen als Ursache für die Aggregation in Frage.

Die der vorliegenden Erfindung zugrund liegende Aufgabe bestand somit in der Bereitstellung von Varianten des Majorallergens Phl p 1 aus Lieschgras, die sich bei vollem Erhalt der therapeutisch und diagnostisch bedeutsamen immunologischen Eigenschaften durch eine verbesserte Löslichkeit auszeichnen und die sich somit in pharmazeutisch geeigneter Form aufreinigen lassen.

### Abbildungen

*Abbildung 1**:* SDS-PAGE des Wildtyps nPhl p 1 (n=natürlich) sowie der Faltungsvarianten LM und HM der Allergenvariante rPhl p 1-A236C (r = rekombinant) unter reduzierenden (in Anwesenheit von Dithiothreitol DTT) und nicht reduzierenden (ohne DTT) Bedingungen.
Bahn 1: Molekulargewichtsstandard (10, 15, 20, 25, 30, 40, 50, 60, 70, 80, 90, 100, 120, 160, 220 kDa, Bench Mark Protein Ladder, Invitrogen, Karlsruhe, Deutschland)
Bahn 2: Extrakt aus *Phleum pratense*-Pollen
Bahn 3: nPhl p 1
Bahn 4: rPhl p 1-LM
Bahn 5: rPhl p 1-HM

*Abbildung 2*: Gelfiltration mit rPhl p 1-HM und rPhl p 1-LM an einer Sephacryl S100-Säule.

Die Abbildung zeigt, daß die beiden Faltungsvarianten unterschiedliche apparente Molekulargewichte aufweisen.

*Abbildung 3*: Enzym-Allergo-Sorbent-Test (EAST) zur Quantifizierung der IgE-Bindung der Faltungsvarianten rPhl p 1-A236C -LM und -HM

Auf der horizontalen Achse ist die Konzentration eines Inhibitors der IgE-nPhl p 1-Bindung in mol/l aufgetragen, auf der vertikalen Achse ist der Grad der Inhibierung in [%] angegeben. Die Messung erfolgte mit nPhl p 1 an der Festphase und einem typischen Serum eines Graspollenallergikers.

### Detaillierte Beschreibung der Erfindung

Überraschenderweise wurde nun gefunden, daß die Einführung eines zusätzlichen Cystein-Restes, vorzugsweise im carboxyterminalen Teil (insbesondere ab Aminosäureposition 140, ganz besondere bevorzugt zwischen den Aminosäurepositionen 230 und 240) des Moleküls zu der erfindungsgemäß verbesserten Löslichkeit bei unveränderter IgE-Aktivität und T-Zell-Reaktivität führt.

Die Erfindung betrifft daher Varianten des Majorallergens Phl p 1 aus Lieschgras, die gegenüber dem Wildtyp einen zusätzlichen Cys-Rest in einer höheren als der Aminosäureposition 140 aufweisen.

Ferner betrifft die Erfindung ein Verfahren zur Herstellung der erfindungsgemäßen Varianten des rekombinanten Majorallergens rPhl p 1, dadurch gekennzeichnet, daß nach an sich bekannten Methoden in das Phl p 1-Gen durch Insertion oder Austausch ein Basentriplett kodierend für einen Cys-Rest eingeführt wird, das so veränderte Gen in einem Wirtsorganismus überexprimiert wird und die durch Überexpression erhaltene Allergenvariante gereinigt wird.

Die prokaryontische rekombinante Herstellung und Aufreinigung kann mit oder ohne einen gentechnisch eingeführten Fusionsanteil durchgeführt werden und führt immer zu gleichen Produkten. Wird ein Fusionsanteil verwendet, handelt es sich dabei vorzugsweise um einen His-Tag. Die Reinigungsverfahren variieren je nach Expressionsvektor bzw. System.

Die vorliegende Erfindung unfaßt demnach eine gezielt veränderte Primärsequenz des rekombinanten Allergens rPhl p 1, die seine rekombinante Herstellung in bakteriellen bzw. anderen Expressionssystemen und die anschließende Reinigung ermöglicht.

Gegenstand der Erfindung sind somit auch DNA-Moleküle, welche für die erfindungsgemäßen Allergenvarianten kodieren.

Die rekombinanten Proteine sind autoproteolytisch inaktiv und können daher in stabiler monomerer Form in physiologischen, gepufferten oder je nach Applikation in anderen Lösungen gelagert werden. Die T-Zell-Stimulierung zeigt keine signifikanten Unterschiede zwischen rekombinantem und natürlichem Phl p 1.

Die rekombinanten Allergenvarianten und die abgeleiteten Fragmente oder Varianten können somit zur Therapie von Graspollen-induzierten allergischen Erkrankungen genutzt werden.

Aufgrund dieser pharmazeutischen Eignung betrifft die vorliegende Erfindung die neuen Allergenvarianten auch in ihrer Eigenschaft als Arzneimittel.

Weiterhin können die rekombinant hergestellten Allergenvarianten und Fragmente zur Diagnostik von Pollenallergien genutzt werden.

Bei der Herstellung des gentechnisch veränderten Majorallergens Phl p 1 aus Lieschgras wird der Aminosäureaustausch durch gerichteten Nukleotidaustausch, beispielsweise mittels PCR, bewirkt. In einer besonders bevorzugten Ausführungsform, der Mutante rPhl p 1-A236C gemäß SEQ ID NO 2, wird das Alanin an Position 236 durch Cystein ausgetauscht. Die Austauschstelle kann jedoch auch an einer beliebigen anderen Stelle des Moleküls liegen. In der Regel wird sie jedoch im C-terminalen Bereich des Moleküls, vorzugsweise ab Position 140, insbesondere zwischen den Positionen 230 und 240 lokalisiert sein.

Als Folge des Austauschs wird gleichzeitig die für Phl p 1 bekannte proteolytische Aktivität eliminiert.

Als ein weiterer unerwarteter Effekt treten bei der erfindungsgemäßen Isolierung und Reinigung des Moleküls zwei Faltungsvarianten auf, die vollständig voneinander getrennt werden können.

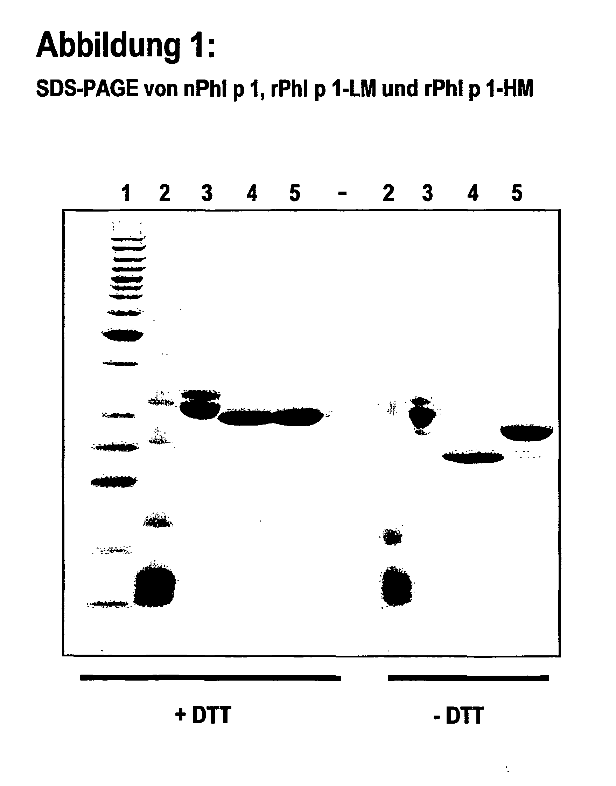

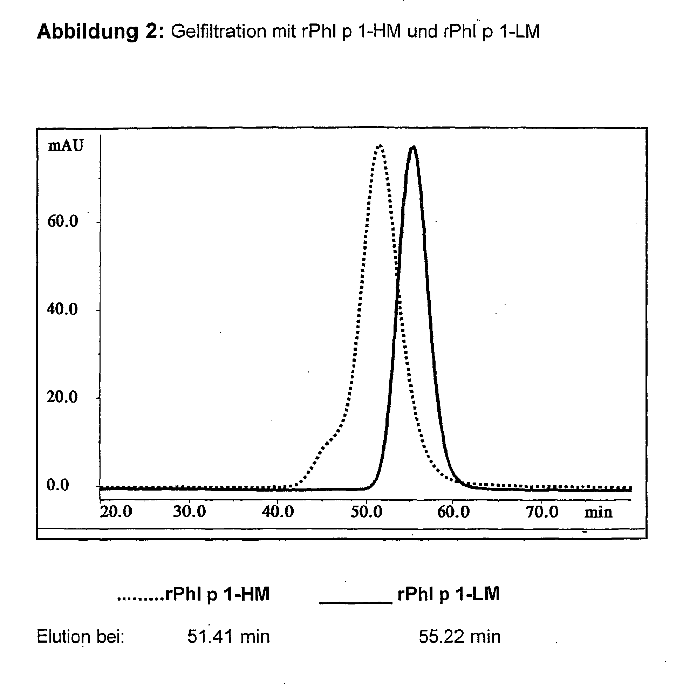

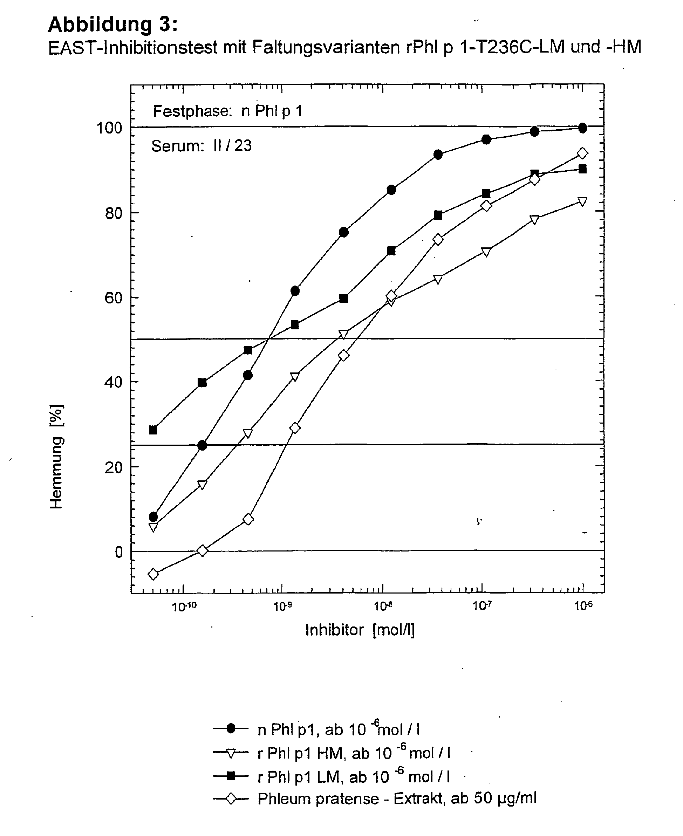

Während sich die eine, als rPhl p 1-LM (LM = low molecular weight) bezeichnete Konformationsvariante aufgrund ihres ähnlichen bzw. identischen Laufverhaltens in der nicht reduzierenden SDS-PAGE (Abb. 1) und der Gelfiltration (bspw. an Sepharcryl S-100, vgl. Abb. 2) zu dem natürlichen Protein sehr ähnlich verhält, liegt die zweite, als rPhl p 1-HM (HM = high molecular weight) bezeichnete Variante in einer anderen Faltungsform vor. Auch die IgE- Reaktivität differiert. Während rPhl p 1-LM eine dem natürlichen Protein vergleichbare Reaktivität aufweist, wird rPhl p 1-HM von IgE-Antikörpern weniger gut gebunden und ist wegen seiner Hypoallergenität in besonderem Maße für die spezifische Immuntherapie geeignet (s. Abb. 3).

Grundsätzlich sind jedoch beide Faltungsformen sowohl für therapeutische als auch diagnostische Anwendungen geeignet.

Gegenstand der Erfindung sind somit weiterhin unterschiedliche Faltungsformen der erfindungsgemäßen rPhl p 1-Allergenvariante und ihre Verwendung für therapeutische und diagnostische Zwecke.

Beide Faltungsvarianten sind leicht löslich, stabil monomer und haben keine nachweisbare proteolytische/autoproteolytische Aktivität.

Die erfindungsgemäßen Allergenvarianten können beispielweise nach den beiden nachfolgend skizzierten Herstellungsverfahren - mit oder ohne artifiziellen Fusionsanteil - erhalten werden:

### 1) Expression mit artifiziellem Fusionsanteil (His-Tag)

Im Fall der Verwendung eines His-Tags erfolgt die Reinigung des zunächst unlöslichen Rohproteins über mehrere biochemische Trennschritte, umfassend einen oder mehrere Metallionen-Chelat-Affinitätschromatographie-Schritte und die Abspaltung des His-Tags. Zur Vor- und Nachreinigung können verschiedene andere Chromatographien sowie De- und Renaturierungsschritte zum Einsatz kommen.

Herstellung der Faltungsform rPhl p 1-LM:
- Denaturierung der aus dem Wirtsorganismus isolierten Einschlußkörper mit Guanidiniumchlorid,
- Renaturierung des gelösten Proteins auf einer Chelat-Affinitätschromatographiesäule,
- Abspalten des His-Tags,
- erste Gelfiltration,
- Chelat-Affinitätschromatographie,
- Isolierung des Zielproteins aus dem Durchlauf, zweite, abschließende Gelfiltration.

Die andere Faltungsform - rPhl p 1-HM - kann bei dieser Reinigungsvariante durch Ausführen der folgenden Verfahrensschritte erhalten werden:
- Denaturierung der aus dem Wirtsorganismus isolierten Einschlußkörper mit Guanidiniumchlorid,
- Renaturierung des gelösten Proteins auf einer Chelat-Affinitätschromatographiesäule,
- Abspalten des His-Tags,
- erste Gelfiltration,
- Chelat-Affinitätschromatographie,
- Elution des Zielproteins mit einem Imidazolgradienten,
- zweite, abschließende Gelfiltration. -

### 2) Expression ohne Fusionsanteil (ohne His-Tag)

- Denaturierung der aus dem Wirtsorganismus isolierten Einschlußkörper mit Guanidiniumchlorid, wobei wie weiter unten beschrieben in Abhängigkeit der Denaturierungsdauer die eine oder die andere Faltungsform erhalten wird.
- Renaturierung des gelösten Proteins durch Verdünnung in Pufferlösung, wobei vorzugsweise 20-50 mM Tris/HCl pH 7-8 zum Einsatz kommt, aber auch andere Puffer und pH-Werte (z.B. 7-10,5) möglich sind. Zur Verdünnung wird bevorzugt der Denaturierungsansatz zu einem Vielfachen seines Volumens (etwa das10 bis 80fache, vorzugsweise das 20 bis 60fache Volumen) der vorzugsweise gerührten oder anderweitig durchmischten Pufferlösung - etwa durch Dekantieren, Pipettieren oder Pumpen - gegeben; jedoch kann auch die Pufferlösung zum Denaturierungsansatz gegeben werden. Die Geschwindigkeit der Zugabe
   ist unkritisch: so kann die gesamte Menge auf einmal innerhalb weniger Sekunden zugegeben werden oder aber - (vorzugsweise jedoch nicht zwingend) gleichmäßig - verteilt auf mehrere Stunden.
- Konzentration und Reinigung des renaturierten Proteins durch Chelat-Affinitätschromatographie und anschließende Elution mit einem Imidazolgradienten (es wird vorzugsweise ein Stufengradient verwendet, ein kontinuierlicher Gradient ist jedoch ebenfalls möglich). Alternativ oder zusätzlich zur Chelat-Affinitätschromatographie kann optional auch eine Hydrophobe-Interaktions-Chromatographie und/oder eine Anionenaustausch-Chromatographie durchgeführt werden (die Moleküle sind vollständig chromatographisch zu bearbeiten).
- abschließende Gelfiltration.

Die alternativen stabilen Faltungsformen LM und HM können hierbei gezielt mit minimaler Kreuzkontamination durch unterschiedliche Inkubationszeiten bei dem Denaturierungsschritt erhalten werden. Zur Herstellung der LM-Form kann dabei grundsätzlich zwischen etwa 1 und 50 Stunden inkubiert werden. In der Regel wird man sich jedoch in einem Bereich von 10 bis 40 Stunden, vorzugsweise 15 bis 30 bewegen, wobei ein Intervall von 18 bis 22 Stunden besonders bevorzugt ist.

Für die HM-Variante werden deutlich längere Inkubationszeiten benötigt. Sie liegen in der Größenordnung von 60 bis 120 Stunden. Für gewöhnlich wird man jedoch 70 bis 100 Stunden inkubieren, besonders bevorzugt bewegt man sich dabei in einem Bereich von 80 bis 90 Stunden.

Auf den Denaturierungsschritt folgt in jedem Fall der zuvor beschriebene Verdünnungsschritt mit Pufferlösung.

Das Zwischenintervall (ca. 50 bis 60 Stunden) repräsentiert die Umbildungsphase. Durch den Verdünnungsschritt wird der Faltungsprozeß offenbar fixiert, es findet keine weitere Kinetik statt.

Die Feinreinigung zur Trennung von LM und HM ist möglich über eine Hydrophobe Interaktionschromatographie oder wird durch Gelfiltration erreicht, bei der die Formen deutlich unterschiedliche Retentionszeiten haben.

Die erfindungsgemäßen Allergenvarianten können in ihren Faltungsvarianten LM und HM in hoher Reinheit erhalten werden und haben wertvolle pharmazeutisch relevante Eigenschaften. So können sie als Gemisch aber auch einzeln zur Diagnostik (insbesondere rPhl p 1-LM wegen des Erhalts der IgE-Aktivität) und Therapie (insbesondere rPhl p 1-HM wegen der verminderten IgE-Aktivität) von allergischen Erkrankungen eingesetzt werden.

Die Erfindung betrifft daher ebenfalls die Verwendung der Allergenvarianten und/oder ihre pharmazeutisch verwendbaren Derivate, einschließlich deren Mischungen in allen Verhältnissen, zur Herstellung eines Arzneimittels zur spezifischen Immuntherapie (Hyposensibilisierung) sowie Diagnostik von Allergien, an deren Auslösung das Majorallergen Phl p 1 aus Lieschgras beteiligt ist

Ferner ist Gegenstand der Erfindung eine pharmazeutische Zubereitung, enthaltend eine erfindungsgemäße Allergenvariante und/oder ihre pharmazeutisch verwendbaren Derivate, einschließlich deren Mischungen in allen Verhältnissen, sowie gegebenenfalls Träger- und/oder Hilfsstoffe. Hierbei können die erfindungsgemäßen Wirkstoffe zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Werden die den erfindungsgemäßen Allergenvarianten zugrunde liegenden DNA-Moleküle mit einem geeigneten Expressionsvektor ligiert, können diese Konstrukte zudem als Präparate für eine Immuntherapie (DNA-Vakzinierung) angewendet werden.

Gegenstand der Erfindung ist daher ebenfalls ein rekombinanter DNA-Expressionsvektor, enthaltend ein erfindungsgemäßes DNA-Molekül, zur Behandlung von Allergien, an deren Auslösung das Majorallergen Phl p 1 aus Lieschgras beteiligt ist, durch immuntherapeutische DNA-Vakzinierung.

Gegenstand der Erfindung ist weiterhin die Verwendung des besagten Expressionsvektors und/oder seiner Derivate, einschließlich deren Mischungen in allen Verhältnissen, zur Herstellung eines Arzneimittels zur Behandlung von Allergien, an deren Auslösung das Majorallergen Phl p 1 aus Lieschgras beteiligt ist, durch immuntherapeutische DNA-Vakzinierung.

Schließlich ist Gegenstand der Erfindung eine pharmazeutische Zubereitung, enthaltend den besagten Expressionsvektor und/oder seine pharmazeutisch verwendbaren Derivate, einschließlich deren Mischungen in allen Verhältnissen, sowie gegebenenfalls Träger- und/oder Hilfsstoffe, zur Behandlung von Allergien, an deren Auslösung das Majorallergen Phl p 1 aus Lieschgras beteiligt ist, durch immuntherapeutische DNA-Vakzinierung.

Pharmazeutische Zubereitungen im Sinne dieser Erfindung können als Therapeutika in der Human- oder Veterinärmedizin verwendet werden. Als Trägerstoffe kommen organische oder anorganische Substanzen in Frage, die sich für die parenterale Applikation eignen und mit erfindungsgemäßen Allergenvarianten des Majorallergens Phl p 1 aus Lieschgras nicht reagieren. Zur parenteralen Anwendung dienen insbesondere Lösungen, vorzugsweise ölige oder wässrige Lösungen, ferner Suspensionen, Emulsionen oder Implantate. Die erfindungsgemäßen Allergenvarianten können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen und/oder mehrere weitere Wirkstoffe enthalten.

Weiterhin können durch entsprechende Formulierung der erfindungsgemäßen Allergenvarianten Depotpräparate, beispielsweise durch Adsorption an Aluminiumhydroxid, erhalten werden.

Naturgemäß sind über die erfindungsgemäßen Mutationen auch weitere punktuelle Veränderungen an anderen Positionen sowie sonstige Modifikationen - etwa zur Erhöhung der Hypoallergenität möglich. Bei diesen Modifikationen kann es sich beispielsweise um chemische Modifikationen des Allergenextrakts handeln (Fiebig, 1995, Allergo J. 4 (7), 377-382). Sie können aber auch gentechnisch auf DNA-Ebene erfolgen, wobei z.B. Aminosäure-Insertionen, -Deletionen und -Austausche, Aufspaltungen des Proteins in Fragmente sowie Fusionen des Proteins oder seiner Fragmente mit anderen Proteinen oder Peptiden in Frage kommen.

Angesichts der hohen Sequenzhomologien innerhalb der Gruppe 1 Graspollenmajorallergene sind sämtliche, hier für Phl p 1 beschriebenen Effekte betreffend die Verbesserung der Löslichkeit durch Einführung eines Cys-Restes und das Auftreten von Faltungsvarianten auch für andere Vertreter dieser Gruppe zu erwarten.

Auch ohne weitere Ausführungen wird davon ausgegangen, dass ein Fachmann die obige Beschreibung in weitestem Umfang nutzen kann. Die nachfolgend beispielhaft für die Variante rPhl p 1-A236C dargestellten Ausführungsformen gemäß der Tabellen 1 und 2 sind deswegen lediglich als beschreibende, keineswegs als in irgendeiner Weise limitierende Offenbarung aufzufassen.

Sämtliche Chromatographiematerialen werden von der Firma Amersham Biosciences (Freiburg, Deutschland) kommerziell vertrieben.

Bei den beschriebenen Chelat-Affinitätschromatographien ist die Wahl des Metallions unkritisch, es kann sowohl Ni als auch Cu verwendet werden.

### Beispiel 1: Gewinnung von rPhl p 1-A236C-LM und -HM

### - Reinigungsvariante mit His-Tag

Die für das Phl p 1 kodierende Sequenz wurde mit 5'- und 3'-spezifischen Oligonukleotiden mittels PCR amplifiziert und in einen pProEx-Vektor (GIBCO, La Jolla, USA) über die Ehe I und Hind III Schnittstelle ligiert. Der 3'-Primer wurde an Basenposition 706/707 von GC nach TG derart verändert, daß aus einem für Alanin kodierendes ein für Cystein kodierendes Triplett entsteht (Essential Molecular Biology; T.A. Brown ed., IRL Press, Oxford, 1994). Die Transformation erfolgte in *E. coli* Origami. Der gewählte Ausgangsvektor pProEx liefert die N-terminalen Ende lokalisierte 6xHis Sequenz, gefolgt von einer Erkennungssequenz für die TEV-Protease.

Die nach der bakteriellen Expression als unlösliche Aggregate vorliegenden rekombinanten, primär 6xHIS getaggten rPhl p 1-A236C-Moleküle werden nach Vorreinigung in 6 M Guanidiumchlorid (GdmCl), 50 mM Tris/HCl, 500 mM NaCl, pH 8.0) gelöst. Es schließt sich eine zweistufige Ni-Chelat-Affinitätschromatographie an:

In einem ersten Schritt werden die unter denaturierenden Bedingungen an Chelating Sepharose gebundenen Proteine durch einen Gradienten über 90 min von der Denaturierungslösung in einen Puffer bestehend aus 50 mM Phosphatpuffer und 500 mM NaCl (pH 7.4) überführt. Es schließt sich eine Stufenelution mit 500 mM Imidiazol in Phosphatpuffer an. Das renaturierte Fusionsprotein wird mittels einer spezifischen TEV-Protease in rPhl p 1 und den 6xHis Fusionsanteil gespalten.

Zur Vorbereitung auf eine zweite Affinitätschromatographie wird eine Gelfiltration mit Sephadex G-25 und Phosphatpuffer als Elutionsmittel durchgeführt, wodurch das Imidiazol entzogen wird.

Das umgepufferte Proteingemisch wird nun zu einer zweiten Ni-Chelat-Affinitätschromatographie eingesetzt. Dabei befindet sich ein Teil des erfolgreich gespaltenen rPhl p 1 im Durchlauf. Es handelt sich hierbei um die LM-Form des Moleküls. An der Säule bleibt neben ungespaltenen Molekülen auch die Konformationsvariante HM haften, offenbar bedingt durch exponierte Histidinreste. Diese gespaltene Variante eluiert in einem Imidazolgradienten eher als die ungespaltenen Fusionsproteine, wodurch auch diese Form hochrein erhalten werden kann. In einem letzten Schritt wird zur Endreinigung und Überführung in ein gewünschtes Lösungsmittel eine Gelfiltration mit Superdex 75 durchgeführt.

**Tab. 1 Übersicht über das erfindungsgemäße Herstellungs- und Reinigungsverfahren unter Verwendung eines His-Tags**

| | |
|---|---|
| 1. Expression | |
| 2. Isolierung der Inclusion Bodies | |
| 3. Denaturierung | |
| 4. Ni-Chelat-Affinitätschromatographie 1 (Renaturierung) | |
| 5. Abspaltung des His-Tags | |
| 6. Gelfiltration (Sephadex G-25) | |
| 7. Ni-Chelat-Affinitätschromatographie 2: | **Durchlauf: rPhl p 1-LM** |
| | **Eluat: rPhl p 1-HM,** ungespaltenes His-rPhl p 1-Fusionsprotein |
| 8. Gelfiltration (Superdex 75) | |

### Beispiel 2: Gewinnung von rPhl p 1-A236C-LM und -HM - Reinigungsvariante ohne His-Tag

Zunächst wird gemäß Beispiel 1 vorgegangen, wobei der gewählte Vektor jedoch als Primärprodukt kein Fusionsprotein liefert.

Nun werden die nach der bakteriellen Expression als unlösliche Aggregate (Inclusion bodies) vorliegenden rekombinanten, primär rPhl p 1-A236C-Moleküle, nach Vorreinigung in 6 M Guanidiumchlorid (GdmCl), 50 mM Tris/HCl, pH 8.0) gelöst. Es schließt sich eine 40fache Verdünnung in 20 mM Tris pH 8.0 an. Die Denaturierungslösung wird dazu in die mit einem Magnetrüher gerührte Verdünnungslösung dekantiert

### a) Herstellung der LM-Form

Die Inclusion bodies werden 20 h in der Denaturierungslösung inkubiert und anschließend wie oben beschrieben verdünnt.

### b) Herstellung der HM-Form

Die Inclusion bodies werden 85 h in der Denaturierungslösung inkubiert und anschließend wie oben beschrieben verdünnt.

Dem jeweiligen Verdünnungs- (Renaturierungs-) Ansatz werden 500 mM NaCl zugesetzt. Die gelösten Moleküle im Renaturierungsansatz werden über eine Cu-Chelat-Affinitätschromatographie konzentriert und mit 200 mM Imidazol in Phosphatpuffer als Stufe elutiert (oder graduell durch 500 mM Imidazol in Phosphatpuffer).

Optional kann die Chelat-Affinitätschromatographie vor der Elution des Proteins zur Konditionierung derart benutzt werden, daß beispielsweise eine 3 M NaCl Lösung zum Waschen und eine 3 M NaCl, 200 mM Imidazollösung zum Eluieren benutzt wird. Das stark salzhaltige Eluat könnte dann direkt für eine Hydrophobe-Interaktions-Chromatographie eingesetzt werden. In einem letzten Schritt wird zur Endreinigung und Überführung in ein gewünschtes Lösungsmittel eine Gelfiltration mit Superdex 75 durchgeführt.

**Tab. 2 Übersicht über das erfindungsgemäße Herstellungs- und Reinigungsverfahren ohne Verwendung eines His-Tags**

| | |
|---|---|
| 1. Expression | |
| 2. Isolierung der Inclusion Bodies | |
| 3. Denaturierung | **Dauer 20 h: LM** |
| | **Dauer 85 h: HM** |
| 4. Verdünnung | |
| 5. Cu-Chelat-Affinitätschromatographie | |
| 6. Gelfiltration (Superdex 75) | |

### Beispiel 2: Unterschiedliche IgE-Bindungen des Wildtyps sowie der Faltungsvarianten LM und HM der Allergenvariante rPhl p 1-A236C

In einem gemäß Suck et al. (Int. Arch. Allergy Immunol. 2000; 121: 284-291) durchgeführten EAST-Inhibitionsassay mit einem Allergiker-Serumpool werden das natürliche nPhl p 1 und die rekombinanten rPhl p.1 Varianten HM sowie LM hinsichtlich der Stärke ihrer IgE-Bindung miteinander verglichen (Abb. 3). Es zeigt sich, daß die Variante HM in ihrer IgE-Bindung gegenüber dem natürlichen Phl p 1 Protein deutlich reduziert ist, während die Variante LM eine mit dem natürlichen Phl p 1 Protein vergleichbare IgE-Bindung aufweist.

## Patentansprüche

1. Variante des Majorallergens Phl p 1 aus Lieschgras, **dadurch gekennzeichnet, dass** sie gegenüber dem Wildtyp einen zusätzlichen Cys-Rest in einer höheren als der Aminosäureposition 140 aufweist, **dadurch gekennzeichnet, dass** der Wildtyp eine Aminosäuresequenz aufweist, die der SEQ ID NO: 2 entspricht, wobei diese Wildtyp-Aminosäuresequenz jedoch im Unterschied zur Aminosäuresequenz gemäß SEQ ID NO: 2 in der Aminosäureposition 236 einen Ala-Rest aufweist.

2. Allergenvariante nach Anspruch 1, **dadurch gekennzeichnet, dass** sich der zusätzliche Cys-Rest zwischen den Aminosäurepositionen 230 und 240 befindet

3. Allergenvariante nach Anspruch 1 oder 2, **dadurch gekennzeichnet, dass** der zusätzliche Cys-Rest aus einem Aminosäureaustausch hervorgegangen ist.

4. Allergenvariante rPhl p 1-A236C gemäß SEQ ID NO: 2 nach einem oder mehreren der Ansprüche 1 bis 3, **dadurch gekennzeichnet, dass** der zusätzliche Cys-Rest durch Austausch von Ala 236 eingeführt wurde.

5. DNA-Molekül, welches für eine Allergenvariante nach einem oder mehreren der Ansprüche 1 bis 4 kodiert.

6. DNA-Molekül gemäß SEQ ID NO 1, welches für die Allergenvariante nach Anspruch 4 kodiert.

7. Verfahren zur Herstellung einer Variante des rekombinanten Majorallergens rPhl p 1 gemäß einem oder mehreren der Ansprüche 1 bis 4, **dadurch gekennzeichnet, dass** nach an sich bekannten Methoden
- in das entsprechende Gen durch Insertion oder Austausch ein Basentriplett kodierend für einen Cys-Rest eingeführt wird,
- das so veränderte Gen in einem Wirtsorganismus überexprimiert wird und
- die durch Überexpression erhaltene Allergenvariante gereinigt wird.

8. Verfahren zur Herstellung und Aufreinigung einer Variante des rekombinanten Majorallergens rPhl p 1 gemäß Anspruch 7 in löslicher Form, **dadurch gekennzeichnet, dass** das zunächst unlösliche Rohprotein denaturiert, anschließend durch Verdünnung renaturiert und durch biochemische Reinigungsschritte gereinigt wird.

9. Verfahren zur Aufreinigung einer Variante des rekombinanten Majorallergens rPhl p 1 gemäß Anspruch 7 in löslicher Form, **dadurch gekennzeichnet, dass** ausgehend von dem zu Reinigungszwecken mit einem His-Tag versehenen überexprimierten, zunächst unlöslichen Rohprotein mehrere biochemische Reinigungsschritte, umfassend eine zweistufige Metallionen-Chelat-Affinitätschromatographien und die Abspaltung des His-Tags, ausgeführt werden.

10. Allergenvariante nach einem oder mehreren der Ansprüche 1 bis 4,
**dadurch gekennzeichnet, dass** sie in unterschiedlichen Faltungsformen vorliegt.

11. Faltungsform rPhl p 1-LM der Allergenvariante gemäß einem oder mehreren der Ansprüche 1 bis 4, erhältlich durch Ausführen der folgenden Verfahrensschritte:
- Überexprimieren der mit einem His-Tag versehenen rPhl p 1-Allergenvariante in einem Wirtsorganismus
- Denaturierung der aus dem Wirtsorganismus isolierten Einschlußkörper mit Guanidiniumchlorid
- Renaturierung des gelösten Proteins auf einer Chelat-Affinitätschromatographiesäule
- Abspalten des His-Tags
- Gelfiltration
- Erneute Chelat-Affinitätschromatographie
- Isolierung des Zielproteins aus dem Durchlauf
- Gelfiltration

12. Faltungsform rPhl p 1-HM der Allergenvariante gemäß einem oder mehreren der Ansprüche 1 bis 4, erhältlich durch Ausführen der folgenden Verfahrensschritte:
- Überexprimieren der mit einem His-Tag versehenen rPhl p 1-Allergenvariante in einem Wirtsorganismus
- Denaturierung der aus dem Wirtsorganismus isolierten Einschlußkörper mit Guanidiniumchlorid
- Renaturierung des gelösten Proteins auf einer Chelat-Affinitätschromatographiesäule
- Abspalten des His-Tags
- Gelfiltration
- Erneute Chelat-Affinitätschromatographie
- Elution des Zielproteins mit einem Imidazolgradienten
- Gelfiltration

13. Allergenvariante nach einem oder mehreren der Ansprüche 1 bis 4 sowie 10 bis 12 als Arzneimittel.

14. Verwendung einer Allergenvariante gemäß Anspruch 13 und/oder ihre pharmazeutisch verwendbaren Derivate, einschließlich deren Mischungen in allen Verhältnissen, zur Herstellung eines Arzneimittels zur spezifischen Immuntherapie von Allergien, an deren Auslösung das Majorallergen Phl p 1 aus Lieschgras beteiligt ist.

15. Pharmazeutische Zubereitung, enthaltend eine Allergenvariante gemäß Anspruch 13 und/oder ihre pharmazeutisch verwendbaren Derivate, einschließlich deren Mischungen in allen Verhältnissen, sowie gegebenenfalls Träger- und/oder Hilfsstoffe.

16. Verwendung einer Allergenvariante nach einem oder mehreren der Ansprüche 1 bis 4 sowie 10 bis 12 und/oder ihrer Derivate, einschließlich deren Mischungen in allen Verhältnissen, zur *in vitro* Diagnostik von Allergien, an deren Auslösung das Majorallergen Phl p 1 aus Lieschgras beteiligt ist.

17. Rekombinanter DNA-Expressionsvektor, enthaltend ein DNA-Molekül nach Anspruch 7 oder 8, zur Behandlung von Allergien, an deren Auslösung das Majorallergen Phl p 1 aus Lieschgras beteiligt ist, durch immuntherapeutische DNA-Vakzinierung.

18. Verwendung des Expressionsvektors nach Anspruch 17 und/oder seiner Derivate, einschließlich deren Mischungen in allen Verhältnissen, zur Herstellung eines Arzneimittels zur Behandlung von Allergien, an deren Auslösung das Majorallergen Phl p 1 aus Lieschgras beteiligt ist, durch immuntherapeutische DNA-Vakzinierung.

19. Pharmazeutische Zubereitung, enthaltend einen Expressionsvektor gemäß Anspruch 17 und/oder seine pharmazeutisch verwendbaren Derivate, einschließlich deren Mischungen in allen Verhältnissen, sowie gegebenenfalls Träger- und/oder Hilfsstoffe, zur Behandlung von Allergien, an deren Auslösung das Majorallergen Phl p 1 aus Lieschgras beteiligt ist, durch immuntherapeutische DNA-Vakzinierung.

## Claims

1. Variant of the major allergen Phl p 1 from Timothy grass, **characterised in that** it has, compared with the wild type, an additional Cys residue in a higher position than amino acid position 140, **characterised in that** the wild type has an amino acid sequence which corresponds to SEQ ID NO: 2, but where this wild-type amino acid sequence has, in contrast to the amino acid sequence according to SEQ ID NO: 2, an Ala residue in amino acid position 236.

2. Allergen variant according to Claim 1, **characterised in that** the additional Cys residue is located between amino acid positions 230 and 240.

3. Allergen variant according to Claim 1 or 2, **characterised in that** the additional Cys residue originates from an amino acid exchange.

4. Allergen variant rPhl p 1-A236C according to SEQ ID NO: 2 according to one or more of Claims 1 to 3, **characterised in that** the additional Cys residue has been introduced by exchange of Ala 236.

5. DNA molecule which encodes for an allergen variant according to one or more of Claims 1 to 4.

6. DNA molecule according to SEQ ID NO: 1 which encodes for the allergen variant according to Claim 4.

7. Process for the preparation of a variant of the recombinant major allergen rPhl p 1 according to one or more of Claims 1 to 4, **characterised in that**, by methods known per se,
- a base triplet encoding for a Cys residue is introduced into the corresponding gene by insertion or exchange,
- the gene modified in this way is overexpressed in a host organism and
- the allergen variant obtained by overexpression is purified.

8. Process for the preparation and purification of a variant of the recombinant major allergen rPhl p 1 according to Claim 7 in soluble form, **characterised in that** the initially insoluble crude protein is denatured, subsequently renatured by dilution and purified by biochemical purification steps.

9. Process for the purification of a variant of the recombinant major allergen rPhl p 1 according to Claim 7 in soluble form, **characterised in that**, starting from the overexpressed, initially insoluble crude protein provided with an His tag for purification purposes, a plurality of biochemical purification steps, encompassing two-stage metal ion chelate affinity chromatography and the removal of the His tag, are carried out.

10. Allergen variant according to one or more of Claims 1 to 4, **characterised in that** it exists in various fold forms.

11. Fold form rPhl p 1-LM of the allergen variant according to one or more of Claims 1 to 4, obtainable by carrying out the following process steps:
- overexpression of the rPhl p 1 allergen variant provided with an His tag in a host organism
- denaturing of the inclusion bodies isolated from the host organism using guanidinium chloride
- renaturing of the dissolved protein on a chelate affinity chromatography column
- removal of the His tag
- gel filtration
- further chelate affinity chromatography
- isolation of the target protein from the flow-through
- gel filtration.

12. Fold form rPhl p 1-HM of the allergen variant according to one or more of Claims 1 to 4, obtainable by carrying out the following process steps:
- overexpression of the rPhl p 1 allergen variant provided with an His tag in a host organism
- denaturing of the inclusion bodies isolated from the host organism using guanidinium chloride
- renaturing of the dissolved protein on a chelate affinity chromatography column
- removal of the His tag
- gel filtration
- further chelate affinity chromatography
- elution of the target protein with an imidazole gradient
- gel filtration.

13. Allergen variant according to one or more of Claims 1 to 4 and 10 to 12 as medicament.

14. Use of an allergen variant according to Claim 13 and/or pharmaceutically usable derivatives thereof, including mixtures thereof in all ratios, for the preparation of a medicament for specific immunotherapy of allergies in the triggering of which the major allergen Phl p 1 from Timothy grass is involved.

15. Pharmaceutical composition comprising an allergen variant according to Claim 13 and/or pharmaceutically usable derivatives thereof, including mixtures thereof in all ratios, and, if desired, excipients and/or adjuvants.

16. Use of an allergen variant according to one or more of Claims 1 to 4 and 10 to 12 and/or derivatives thereof, including mixtures thereof in all ratios, for the *in vitro* diagnosis of allergies in the triggering of which the major allergen Phl p 1 from Timothy grass is involved.

17. Recombinant DNA expression vector containing a DNA molecule according to Claim 5 or 6 for the treatment of allergies in the triggering of which the major allergen Phl p 1 from Timothy grass is involved, by immunotherapeutic DNA vaccination.

18. Use of the expression vector according to Claim 17 and/or derivatives thereof, including mixtures thereof in all ratios, for the preparation of a medicament for the treatment of allergies in the triggering of which the major allergen Phl p 1 from Timothy grass is involved, by immunotherapeutic DNA vaccination.

19. Pharmaceutical composition comprising an expression vector according to Claim 17 and/or pharmaceutically usable derivatives thereof, including mixtures thereof in all ratios, and, if desired, excipients and/or adjuvants, for the treatment of allergies in the triggering of which the major allergen Phl p 1 from Timothy grass is involved, by immunotherapeutic DNA vaccination.

## Revendications

1. Variante de l'allergène majeur Phl p 1 de la fléole des prés, **caractérisée en ce qu'**elle comporte, par comparaison avec le type sauvage, un résidu additionnel Cys en une position plus haute qu'une position d'acide aminé 140, **caractérisée en ce que** le type sauvage comporte une séquence d'acides aminés qui correspond à SEQ ID NO: 2, mais où cette séquence d'acides aminés de type sauvage comporte, par contraste avec la séquence d'acides aminés selon SEQ ID NO: 2, un résidu Ala à la position d'acide aminé 236.

2. Variante d'allergène selon la revendication 1, **caractérisée en ce que** le résidu additionnel Cys est localisé entre les positions d'acide aminé 230 et 240.

3. Variante d'allergène selon la revendication 1 ou 2, **caractérisée en ce que** le résidu additionnel Cys prend son origine à partir d'un échange d'acides aminés.

4. Variante d'allergène rPhl p 1-A236C selon SEQ ID NO: 2 selon une ou plusieurs des revendications 1 à 3, **caractérisée en ce que** le résidu additionnel Cys a été introduit par échange d'Ala 236.

5. Molécule d'ADN qui code pour une variante d'allergène selon une ou plusieurs des revendications 1 à 4.

6. Molécule d'ADN selon SEQ ID NO: 1 qui code pour la variante d'allergène selon la revendication 4.

7. Procédé pour la préparation d'une variante de l'allergène majeur recombinant rPhl p 1 selon une ou plusieurs des revendications 1 à 4, **caractérisé en ce que**, au moyen de procédés connus en soi,
- un triplet de base codant pour un résidu Cys est introduit dans le gène correspondant par insertion ou échange,
- le gène modifié de cette façon est sur-exprimé dans un organisme hôte et
- la variante d'allergène obtenue par sur-expression est purifiée.

8. Procédé pour la préparation et la purification d'une variante de l'allergène majeur recombinant rPhl p 1 selon la revendication 7 sous forme soluble, **caractérisé en ce que** la protéine brute initialement insoluble est dénaturée, est ensuite renaturée par dilution et est purifiée au moyen d'étapes de purification biochimique.

9. Procédé pour la purification d'une variante de l'allergène majeur recombinant rPhl p 1 selon la revendication 7 sous forme soluble, **caractérisé en ce que**, en partant de la protéine brute initialement insoluble surexprimée munie d'une étiquette His à des fins de purification, une pluralité d'étapes de purification biochimique, englobant une chromatographie par affinité chélate ion métal à deux niveaux et l'enlèvement de l'étiquette His, sont mises en oeuvre.

10. Variante d'allergène selon une ou plusieurs des revendications 1 à 4, **caractérisée en ce qu'**elle existe selon diverses formes de repliement.

11. Forme repliée rPhl p 1-LM de la variante d'allergène selon une ou plusieurs des revendications 1 à 4, pouvant être obtenue en mettant en oeuvre les étapes de processus qui suivent :
- sur-expression de la variante d'allergène rPhl p 1 munie d'une étiquette His dans un organisme hôte
- dénaturation des corps d'inclusion isolés à partir de l'organisme hôte en utilisant du chlorure de guanidinium
- renaturation de la protéine dissoute sur une colonne de chromatographie par affinité chélate
- enlèvement de l'étiquette His
- filtration par gel
- chromatographie par affinité chélate supplémentaire
- isolation de la protéine cible à partir du flux continu
- filtration par gel.

12. Forme repliée rPhl p 1-HM de la variante d'allergène selon une ou plusieurs des revendications 1 à 4, pouvant être obtenue en mettant en oeuvre les étapes de processus qui suivent :
- sur-expression de la variante d'allergène rPhl p 1 munie d'une étiquette His dans un organisme hôte
- dénaturation des corps d'inclusion isolés à partir de l'organisme hôte en utilisant du chlorure de guanidinium
- renaturation de la protéine dissoute sur une colonne de chromatographie par affinité chélate
- enlèvement de l'étiquette His
- filtration par gel
- chromatographie par affinité chélate supplémentaire
- élution de la protéine cible avec un gradient d'imidazole
- filtration par gel.

13. Variante d'allergène selon une ou plusieurs des revendications 1 à 4 et 10 à 12 en tant que médicament.

14. Utilisation d'une variante d'allergène selon la revendication 13 et/ou de ses dérivés pharmaceutiquement utilisables, y compris des mélanges afférents selon toutes proportions, pour la préparation d'un médicament pour une immunothérapie spécifique d'allergies au niveau du déclenchement desquelles l'allergène majeur Phl p 1 de la fléole des prés est mis en jeu.

15. Composition pharmaceutique comprenant une variante d'allergène selon la revendication 13 et/ou ses dérivés pharmaceutiquement utilisables, y compris des mélanges afférents selon toutes proportions, et, si souhaité, des excipients et/ou adjuvants.

16. Utilisation d'une variante d'allergène selon une ou plusieurs des revendications 1 à 4 et 10 à 12 et/ou de ses dérivés, y compris des mélanges afférents selon toutes proportions, pour le diagnostic *in vitro* d'allergies au niveau du déclenchement desquelles l'allergène majeur Phl p 1 de la fléole des prés est mis en jeu.

17. Vecteur d'expression d'ADN recombinant contenant une molécule d'ADN selon la revendication 5 ou 6 pour le traitement d'allergies au niveau du déclenchement desquelles l'allergène majeur Phl p 1 de la fléole des prés est mis en jeu, par vaccination d'ADN immunothérapeutique.

18. Utilisation du vecteur d'expression selon la revendication 17 et/ou de ses dérivés, y compris des mélanges afférents selon toutes proportions, pour la préparation d'un médicament pour le traitement d'allergies au niveau du déclenchement desquelles l'allergène majeur Phl p 1 de la fléole des prés est mis en jeu, par vaccination ADN immunothérapeutique.

19. Composition pharmaceutique comprenant un vecteur d'expression selon la revendication 17 et/ou ses dérivés pharmaceutiquement utilisables, y compris des mélanges afférents selon toutes proportions, et, si souhaité, des excipients et/ou adjuvants, pour le traitement d'allergies au niveau du déclenchement desquelles l'allergène majeur Phl p 1 de la fléole des prés est mis en jeu, par vaccination ADN immunothérapeutique.


### SEQUENCE LISTING

<110> Merck Patent GmbH
<120> Varianten des Majorallergens Phl p 1 aus Lieschgras
<130> P 02/129
<140> EP 02 018 157.4
   <141> 2002-08-19
<160> 2
<170> PatentIn version 3.1
<210> 1
   <211> 723
   <212> DNA
   <213> Phleum pratense
<220>
   <221> CDS
   <222> (1)..(720)
   <223>
<400> 1
   atc ccc aag gtt ccc ccc ggc ccg aac atc acg gcg acc tac ggc ggc      48
   Ile Pro Lys Val Pro Pro Gly Pro Asn Ile Thr Ala Thr Tyr Gly Gly
   1               5                   10                  15
   aag tgg ctg gac gcg aag agc acc tgg tac ggc aag ccg acg gcc gcc      96
   Lys Trp Leu Asp Ala Lys Ser Thr Trp Tyr Gly Lys Pro Thr Ala Ala
               20                  25                  30
   ggt ccc aag gac aac ggc ggc gcg tgc ggg tac aag gac gtg gac aag     144
   Gly Pro Lys Asp Asn Gly Gly Ala Cys Gly Tyr Lys Asp Val Asp Lys
           35                  40                  45
   ccc ccg ttc agc ggc atg acc ggc tgc ggc aac acc ccc atc ttc aag     192
   Pro Pro Phe Ser Gly Met Thr Gly Cys Gly Asn Thr Pro Ile Phe Lys
       50                  55                  60
   tcc ggc cgg ggc tgc ggc tcc tgc ttc gag atc aag tgc acc aag ccc     240
   Ser Gly Arg Gly Cys Gly Ser Cys Phe Glu Ile Lys Cys Thr Lys Pro
   65                  70                  75                  80
   gag gcc tgc tcc ggc gag ccc gtg gtg gtc cac atc acc gac gac aac     288
   Glu Ala Cys Ser Gly Glu Pro Val Val Val His Ile Thr Asp Asp Asn
                   85                  90                  95
   gag gag ccc atc gcc gcg tac cac ttc gac ctc tcc ggc atc gcg ttc     336
   Glu Glu Pro Ile Ala Ala Tyr His Phe Asp Leu Ser Gly Ile Ala Phe
               100                 105                 110
   ggg tcc atg gcc aag aag ggc gac gag cag aag ctg cgc agc gcc ggc     384
   Gly Ser Met Ala Lys Lys Gly Asp Glu Gln Lys Leu Arg Ser Ala Gly
           115                 120                 125
   gag gtg gag atc cag ttc cgc cgc gtc aag tgc aag tac ccg gag ggc     432
   Glu Val Glu Ile Gln Phe Arg Arg Val Lys Cys Lys Tyr Pro Glu Gly
       130                 135                 140
   acc aag gtg acc ttc cac gtg gag aag ggg tcc aac ccc aac tac ctg     480
   Thr Lys Val Thr Phe His Val Glu Lys Gly Ser Asn Pro Asn Tyr Leu
   145                 150                 155                 160
   gcg ctg ctg gtg aag ttt gtc gcc ggc gac ggc gac gtg gtg gcg gtg     528
   Ala Leu Leu Val Lys Phe Val Ala Gly Asp Gly Asp Val Val Ala Val
                   165                 170                 175
   gac atc aag gag aag ggc aag gac aag tgg atc gcg ctc aag gag tcg     576
   Asp Ile Lys Glu Lys Gly Lys Asp Lys Trp Ile Ala Leu Lys Glu Ser
               180                 185                 190
   tgg gga gcc atc tgg agg atc gac acc ccg gag gtg ctc aag ggc ccc     624
   Trp Gly Ala Ile Trp Arg Ile Asp Thr Pro Glu Val Leu Lys Gly Pro
           195                 200                 205
   ttc acc gtc cgc tac acc acc gag ggc ggc acc aag ggc gag gcc aag     672
   Phe Thr Val Arg Tyr Thr Thr Glu Gly Gly Thr Lys Gly Glu Ala Lys
       210                 215                 220
   gac gtc atc ccc gag ggc tgg aag gcc gac acc tgc tac gag tcc aag     720
   Asp Val Ile Pro Glu Gly Trp Lys Ala Asp Thr Cys Tyr Glu Ser Lys
   225                 230                 235                 240
   tga                                                                 723
<210> 2
   <211> 240
   <212> PRT
   <213> Phleum pratense
<400> 2
   Ile Pro Lys Val Pro Pro Gly Pro Asn Ile Thr Ala Thr Tyr Gly Gly
   1               5                   10                  15
   Lys Trp Leu Asp Ala Lys Ser Thr Trp Tyr Gly Lys Pro Thr Ala Ala
               20                  25                  30
   Gly Pro Lys Asp Asn Gly Gly Ala Cys Gly Tyr Lys Asp Val Asp Lys
           35                  40                  45
   Pro Pro Phe Ser Gly Met Thr Gly Cys Gly Asn Thr Pro Ile Phe Lys
       50                  55                  60
   Ser Gly Arg Gly Cys Gly Ser Cys Phe Glu Ile Lys Cys Thr Lys Pro
   65                  70                  75                  80
   Glu Ala Cys Ser Gly Glu Pro Val Val Val His Ile Thr Asp Asp Asn
                   85                  90                  95
   Glu Glu Pro Ile Ala Ala Tyr His Phe Asp Leu Ser Gly Ile Ala Phe
               100                 105                 110
   Gly Ser Met Ala Lys Lys Gly Asp Glu Gln Lys Leu Arg Ser Ala Gly
           115                 120                 125
   Glu Val Glu Ile Gln Phe Arg Arg Val Lys Cys Lys Tyr Pro Glu Gly
       130                 135                 140
   Thr Lys Val Thr Phe His Val Glu Lys Gly Ser Asn Pro Asn Tyr Leu
   145                 150                 155                 160
   Ala Leu Leu Val Lys Phe Val Ala Gly Asp Gly Asp Val Val Ala Val
                   165                 170                 175
   Asp Ile Lys Glu Lys Gly Lys Asp Lys Trp Ile Ala Leu Lys Glu Ser
               180                 185                 190
   Trp Gly Ala Ile Trp Arg Ile Asp Thr Pro Glu Val Leu Lys Gly Pro
           195                 200                 205
   Phe Thr Val Arg Tyr Thr Thr Glu Gly Gly Thr Lys Gly Glu Ala Lys
       210                 215                 220
   Asp Val Ile Pro Glu Gly Trp Lys Ala Asp Thr Cys Tyr Glu Ser Lys
   225                 230                 235                 240